# EUROPÄISCHE PATENTSCHRIFT

(11) **EP 0 409 113 B1**
(45) Veröffentlichungstag und Bekanntmachung des Hinweises auf die Patenterteilung: **12.06.1996**
(21) Anmeldenummer: 90113505.3
(22) Anmeldetag: 14.07.1990
(51) Int. Cl.: C12N 15/16, A61K 38/08, C12P 21/02, C07K 14/00

(54) **Muteine des menschlichen Erythropoetins, ihre Herstellung und ihre Verwendung**
Human erythropoietin muteins, their production and their use
Mutéines de l'érythropoiétine humaine, leur fabrication et leur utilisation

(30) Priorität: 20.07.1989 DE 3923963
(43) Veröffentlichungstag der Anmeldung: 23.01.1991
(73) Patentinhaber: BEHRINGWERKE Aktiengesellschaft, 35001 Marburg (DE)
(72) Erfinder: Fibi, Mathias, Dr., D-3550 Marburg (DE); Zettlmeissl, Gerd, Dr., D-3551 Lahntal (DE); Küpper, Hans, Dr., D-3550 Marburg (DE)

(56) Entgegenhaltungen:
- EP-A- 0 148 605
- EP-A- 0 357 804
- US-A- 4 835 260
- JOURNAL OF BIOLOGICAL CHEMISTRY, Band 263, Nr. 33, 25 November 1988, Baltimore, MD (US); S. DUBE et al., Seiten 17516-17521

## Beschreibung

Die Anmeldung betrifft ein Mutein des menschlichen Erythropoetins (EPO), das mit Hilfe von DNA-Rekombinationstechniken hergestellt wird und gegenüber dem menschlichen Wildtyp-Erythropoetin vorteilhafte Eigenschaften aufweist.

Reifes menschliches Erythropoetin ist ein Glykoprotein mit einem Molekulargewicht von 34 - 38 kD. Das reife Protein besteht aus 166 Aminosäuren (AS) und der Anteil der Glykosylreste am Molekulargewicht beträgt ca. 40 % (Jacobs et al., (1985), Nature 313, 806-809; Dordal et al., (1985), Endocrinology 116, 2293-2299).

Die biologische Funktion von EPO besteht darin, die Bereitstellung von Erythrozyten zu gewährleisten. EPO stimuliert dabei sowohl Differenzierungs- als auch Teilungsvorgänge in erythroiden Vorläuferzellen.

Das Gen für menschliches Erythropoetin konnte aus einer fötalen Leber-Genbank isoliert und charakterisiert werden und steht seit 1985 für gentechnische Untersuchungen zur Verfügung (Jacobs et al., a.a.O.). Erythropoetin kann mit Hilfe von DNA-Rekombinationstechniken in Animalzellen exprimiert werden und ermöglicht unter anderem eine Therapie der renalen Anämie. Erste therapeutische Erfahrungen mit Wildtyp-EPO zeigten, daß die Erfolgsquote bei behandelten Patienten zwar sehr hoch liegt, zeigten aber auch, daß teilweise grenzwertige Blutdrucke und Blutviskositäten erreicht wurden. So war der Anstieg des Hämatokrits, des Hämoglobins und der Zahl der Vorläuferzellen (Burst-forming Unit erythroide Zellen, BFU-E) in manchen Patienten sehr drastisch, wobei es wünschenswert wäre, einen moderateren Anstieg zu haben. Bei anderen Patienten war der Anstieg zu schwach und es wäre wünschenswert, einen deutlicheren Anstieg der Blutwerte zu erreichen. Eine unphysiologische Erhöhung der Dosis bei schwach ansprechenden Patienten ist wegen provozierbarer Immunreaktionen kontraindiziert. Langzeittherapien werden dadurch erschwert oder sogar verhindert.

Die unterschiedlichen Reaktionen der Patienten beruhen mit hoher Wahrscheinlichkeit auf den jeweils individuellen Fähigkeiten, die EPO-Gaben zu regulieren. EPO hat deshalb bei verschiedenen Patienten individuelle, unterschiedliche Therapieverläufe.

Von besonderer Bedeutung bei der Wechselwirkung des Hormons mit dem Organismus ist neben der Struktur des Proteinanteils die Struktur der Zuckerseitenketten des Moleküls. Beispielsweise zeigt desialyliertes EPO nach Applikation im Tier keine Wirkung. Trotzdem bindet es noch an den Rezeptor und stimuliert Vorläuferzellen. Der Aktivitätsverlust in vivo von Asialo-EPO kann dadurch erklärt werden, daß dieses über Rezeptoren mit Spezifität für Galaktosylreste, die in desialyliertem EPO zugänglich sind, in der Leber entfernt wird. Selbst vollkommen deglykosyliertes EPO zeigt in vitro noch Bindungsaktivität an die Zielzellen, wird aber in vivo schneller über einen noch unbekannten Mechanismus in der Niere ausgeschieden. Die EPO-Bindungsstelle für den Rezeptor wird also durch eine Deglykosylierung nicht verändert. Die verminderte Wirkung in vivo deutet jedoch darauf hin, daß die vollständige Glykosylierung und Sialylierung beim Transport im Blut, bei der Stabilität und für die Eliminierungsgeschwindigkeit aus dem System bedeutend sind.

Das bisher therapeutisch eingesetzte Wildtyp-EPO bewirkt bei manchen Patienten eine Erhöhung des Blutdruckes, was in der Therapie ein Nachteil ist. Es ist anzunehmen, daß EPO in die Blutdruckregulierung eingebunden ist. Deshalb wäre es wünschenswert, Proteine mit der physiologischen Wirkung des EPO zur Verfügung zu haben, die diese negativen Eigenschaften nicht aufweisen, aber nach wie vor die Differenzierung und Teilungsrate von Vorläuferzellen zu Erythrozyten stimulieren.

Eine weitere Nebenwirkung von EPO, die sich bei einigen Patienten zeigt, ist die Stimulierung der Megakaryozyten zur Bildung von Thrombozyten. Dabei kann es während der Therapie mit EPO zu Thrombosegefahr kommen, und die Therapie muß sofort abgesetzt werden. Wünschenswert wäre hier eine höhere Spezifität des eingesetzten Erythropoetins.

Der Erfindung liegt die Aufgabe zugrunde, ein Mutein des EPO bereitzustellen, welches Vorteile gegenüber Wildtyp-EPO besitzt. Das Mutein soll vorzugsweise eine erhöhte biologische Aktivität besitzen.

Wir haben gefunden, daß die gestellte Aufgabe überraschenderweise durch das Mutein EPO (GIn 24), worin die Aminosäure Asn 24 durch die Aminosäure GIn 24 ersetzt ist, des menschlichen Erythropoetins gelöst wird, da dieses Mutein günstigere biologische Eigenschaften als natürliches EPO aufweist.

Die vorliegende Erfindung betrifft daher ein Arzneimittel, enthaltend das Mutein EPO (GIn 24) des menschlichen Erythropoetins, wobei die Aminosäure Asn 24 des Wildtypmoleküls durch GIn 24 ersetzt ist und einen physiologischen verträglichen Träger.

Die Erfindung betrifft außerdem die Verwendung des Muteins EPO (GIn 24) des menschlichen Eryhropoetins, worin die Aminosäure Asn 24 durch GIn 24 ersetzt ist, zur Herstellung eines Arzneimittels zur Behandlung der renalen Anämie und zur Anwendung bei Therapien, die eine Erhöhung der Erythrozytenzahl und/oder eine Erhöhung der Erythrozytenqualität zum Ziel haben.

Die Erfindung ist schließlich in den Beispielen und den Patentansprüchen enthalten.

### Beispiele

### 1. Herstellung einer EPO Mutante (Allgemeine Methoden)

### 1) Synthese eines EPO-spezifischen Oligonukleotids

Mutagenese-Oligonukleotid und Sequenzier-Primer wurden nach der Phosphat-Triester-Methode (Letsinger, (1975), J. Amer. Chem. Soc. 97, 3278 und dto. (1976) J. Amer. Chem. Soc. 98, 3655) hergestellt. Das für die Mutante EPO (GIn 24) verwendete Mutageneseoligonukleotid hat die folgende Sequenz:

5' gcc cgt cgt gat ctg ctc ggc ctc c 3'    EPO (Gln 24).

### 2) Klonierung der EPO c-DNA in das Mutagenese Vektorsystem

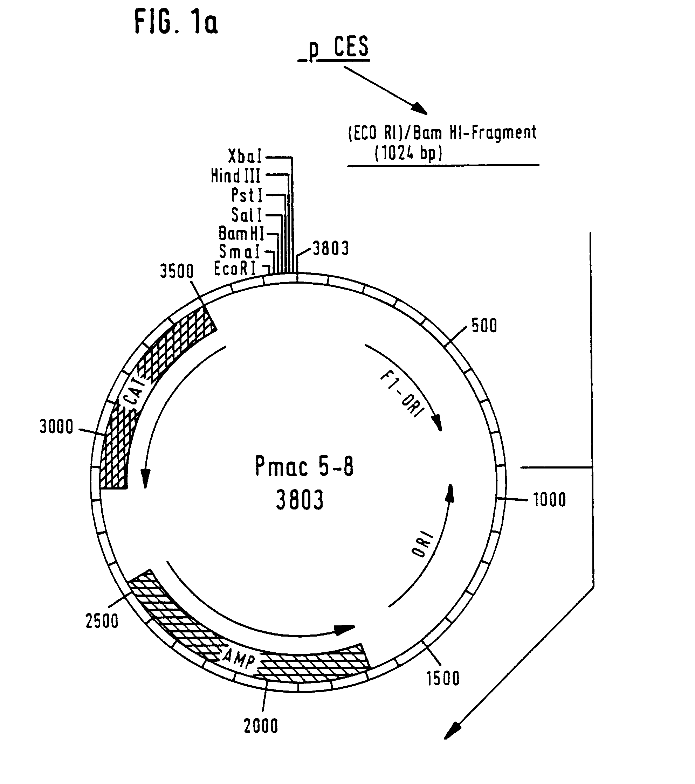

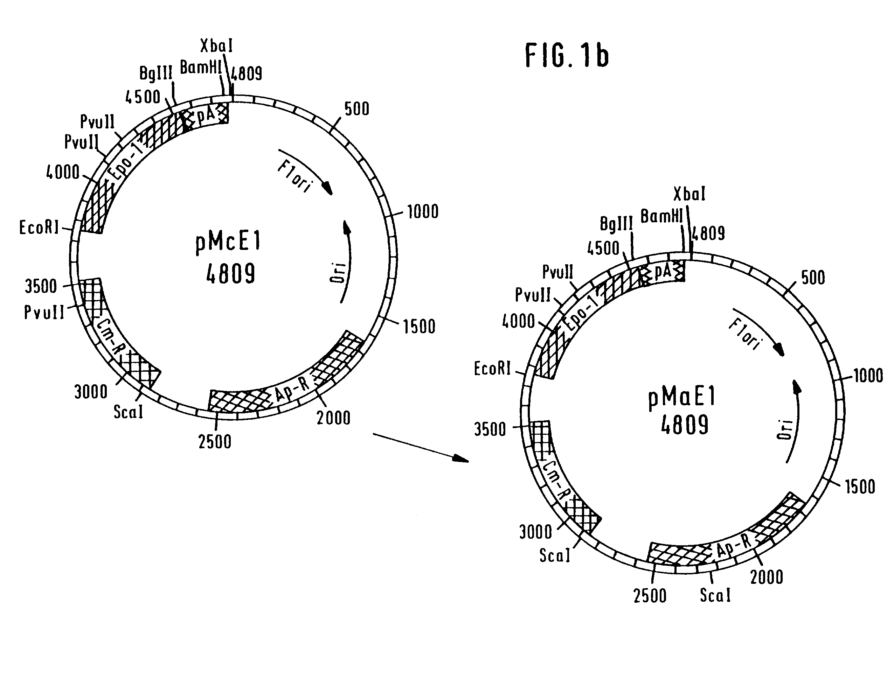

Aus dem Vektor pEPO 782 MT BPV (pCES, EP-A-267 678) wurde das EcoRI-BamHI Fragment von 1024 bp isoliert, welches die EPO-kodierende Sequenz und 3' davon ein SV40 DNA-Fragment mit dem Polyadenylierungssignal des SV40 major late Antigens enthält (Fig. 1). Die Isolierung des Fragments wurde dadurch erreicht, daß das Plasmid mit der Restriktionsendonuklease EcoRI gespalten und die überhängenden Enden mit Hilfe des Klenow-Fragments der DNA-Polymerase I aufgefüllt wurde, so daß ein stumpfes Ende entsteht. Das so behandelte Plasmid wurde mit dem Restriktionsenzym BamHI nachgeschnitten und das oben beschriebene DNA-Fragment konnte dann durch Elution aus einem Agarosegel isoliert werden (Maniatis et al. (1982) Molecular Cloning - A Laboratory Manual, Cold Spring Harbor, New York).

Das Fragment wurde dann in den mit Smal-BamHI geschnittenen Polylinker des Mutagenesevektors pMac 5-8 (Fig. 1) gerichtet kloniert. Diese EPO-Wildtypenkonstruktion wurde pMcE1 genannt.

### 3) Mutagenese

Bei der Herstellung der Mutante EPO (GIn 24) wurde ein synthetisches Oligonukleotid, das die Mutation enthielt (siehe oben), über ein sogenanntes "gapped duplex" DNA Hybridmolekül in das Mutagenese Vektorsystem eingeführt (Morinaga et al., Bio Technology, (1984), 7, 636-639; Kramer et al., Nucl. Acids. Res. 1984, 12, 9441-9456). Dazu wurde Einzelstrang-DNA des Mutagenesevektors pMcE1, welcher in den E.coli-Stamm WK6 transformiert worden war, nach Standardmethoden isoliert. Plasmid-DNA von pMa 5-8 wurde mit EcoR1-BamHI im Polylinker geschnitten und die linearisierte DNA (3.8 kb) aus einem Agarosegel eluiert (Maniatis et al., a.a.O.).

Zur Herstellung einer "gapped duplex" DNA wurden 0.1 pmol doppelsträngiges Fragment (aus pMA 5-8) (Fig. 1) und 0.5 pmol Einzelstrang DNA (pMcE1) (Fig. 1) in 12.5 mM Tris-HCL, pH 7.5 + 190 mM KCI (Endvolumen 40 µl) für 4 Min. bei 100°C erhitzt und anschließend 10 Min. bei 65°C inkubiert. Zur Anhybridisierung des Mutageneseoligonukleotids wurden 8 µl der genannten Hybridisierungslösung mit 4-8 pmol (2 µl) des enzymatisch phosphorylierten Oligonukleotids für 5 Minuten auf 65°C erhitzt und dann langsam auf Raumtemperatur abgekühlte Nach Zugabe von 24 µl H₂O, 4 µl 10 x "fill in" Puffer (625 mM KCI, 275 mM Tris-HCL pH 7.5, 150 mM MgC12, 20 mM DTT, 0.5 mM ATP und je 0.25 mM der vier NTP's), 1 µl T4 DNA-Ligase (5 U/µl) und 1 µl Klenow-Fragment der DNA-Polymerase I (1 U/µl) folgte eine 45-minütige Inkubation bei Raumtemperatur. Anschließend wurden 5 µl des Ansatzes in WK6 muts (mutS215: Tn10) transformiert. Der gesamte Transformationsansatz wurde in einer Schüttelkultur in LB Medium + 50 µg/ml Ampicillin (10 ml) über Nacht bei 37°C vermehrt. Aus dem gesamten Ansatz wurde die Plasmid-DNA nach Standardmethoden gereinigt (Maniatis et al., a.a.O.).

Etwa 20 ng des gereinigten Plasmids wurden in WK6 Bakterien transformiert und anschließend auf LB Platten mit 50 µg/ml Amplicillin selektioniert. Mehrere dieser Mutanten wurden zunächst grob durch eine geeignete Sequenzreaktion (C-, T-, A- oder G-spezifisch) auf die gewünschte Mutation analysiert. Positive Klone wurden durch detaillierte Sequenzanalyse im Berich der Mutagenese bestätigt (F. Sanger et al., (1977), Proc. Natl. Acad. Sci, USA 74, 5463-5467). Plasmide, die mutierte EPO-Sequenzen enthielten, wurden mit pMaE2, pMaE3... pMaEn bezeichnet (Fig. 1).

### 4) Konstruktion eines Expressionsvektors für EPO und die EPO-Mutante EPO (GIn 24)

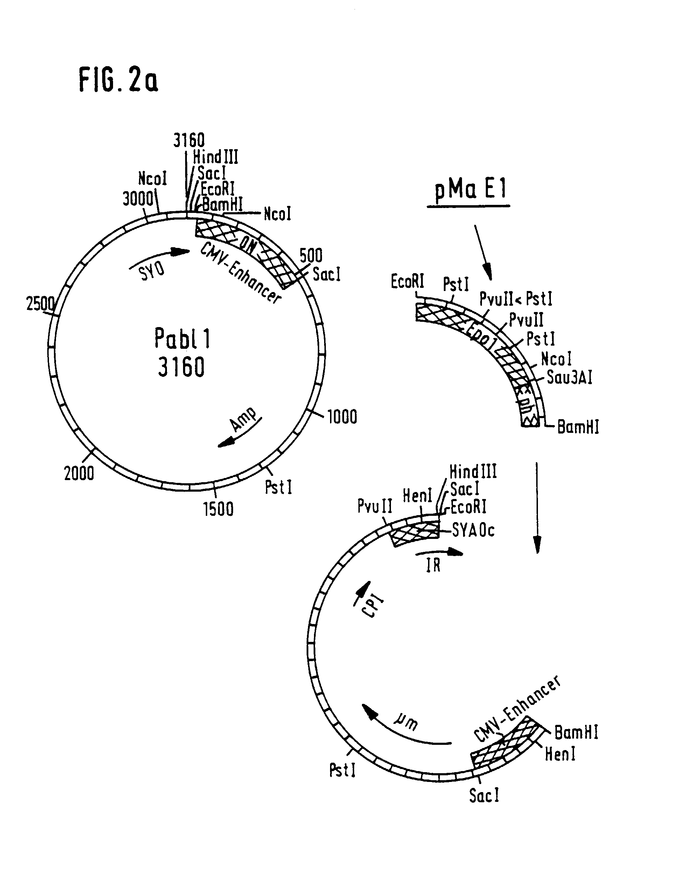

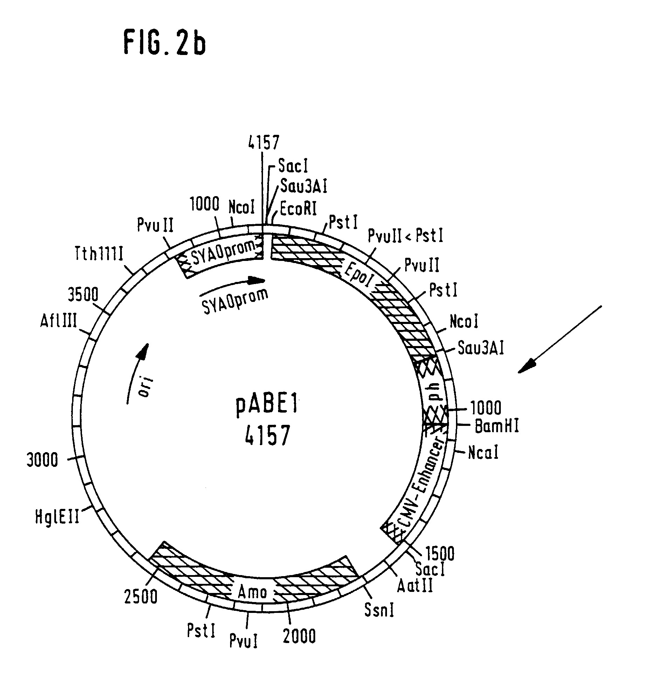

Der Expressionsvektor pABL1 (Fig. 2) wurde dadurch hergestellt, daß aus dem Expressionsvektor pAB 3-1 (Zettlmeißl et al., (1988), Behring Inst. Mitt. 82, 26-34) das für Antithrombin III kodierende Fragment sowie das Fragment mit der early Polyadenylierungsstelle von SV40 durch Schneiden mit den Restriktionsenzymen Hindlll und BamHI entfernt wurde und durch einen Polylinker folgender Sequenz ersetzt wurde:

5' gga tcc ccg ggt acc gag ctc gaa ttc atc gat atc tag atc tcg agc tcg cga aag ctt 3'

BamHI EcoRI SaCI HindIII

Das EPO (wt) und EPO (Gln 24) kodierende Fragment konnte dann aus dem Mutagenesevektorsystem mit EcoRI - BamHI herausgeschnitten werden und dann, ebenfalls über EcoRI und BamHI Schnittstellen des Polylinkers, in den Expressionsvektor pABL1 kloniert werden.

### 5) Transfektion von Animalzellen und Doppelselektion

BHK21-Zellen (baby hamster kidney) (ATCCCCL10) wurden mit dem EPO (wt)-kodierenden oder mit dem EPO (GIn 24) kodierenden Expressionsvektor transfiziert. Zur Transfektion wurden die Zellen in "Dulbecco's modified Eagle's medium" (DMEM) gezüchtet, welches 10 % fötales Kälberserum enthielt. Bei 50-70 % Konfluenz wurden die Zellen mit Hilfe einer modifizierten Kalziumphosphatmethode transfiziert (Graham and van der Eb, (1973), Virology, 52, 456-467).

Die Expressionsvektoren wurden kotransfiziert mit dem Plasmid pSV2 dhfr (Lee et al., (1981), Nature (London), 294, 228-232), welches ein in Animalzellen exprimierbares Maus-Dihydrofolatreduktase (dhfr) Gen enthält und mit Plasmid pRMH140 (Hudziak et al., (1982), Cell, 31, 137-146), welches eine in Animalzellen exprimierbare Geneticin Resistenz enthält. Dies System erlaubt eine Doppelselektion mit Methotrexat (1 mM) und Geneticin (G418, 400 µg/ml) und ermöglicht eine Amplifikation der ins zelluläre Genom integrierten Plasmid DNA's (Zettlmeißl et al., a.a.O.).

### 6) Immunologischer Nachweis von EPO und EPO Muteinen aus Kulturüberständen exprimierender Zellen Herstellung von Antiseren gegen EPO

Gegen gereinigtes EPO wurden Antikörper in Kaninchen hergestellt. Zu diesem Zweck wurde EPO mit Hilfe von Glutardialdehyd an "Keyhole Limpet" Hämocyanin (KLH) gekoppelt, mit Adjuvantien emulgiert und als Emulgat immunisiert. Die Gewinnung der Seren erfolgte nacht Standardmethoden.

### Radioimmunoassay zur Messung von Erythropoetin

Dieser Test dient zur quantitativen Bestimmung von rekombinantem EPO in Zellkulturüberständen und in Proben aus verschiedenen Stufen der Reinigung des Hormons. Er basiert auf der Doppelantikörper-Variante des kompetitiven Radioimmunassays (C.N. Hales, P.J. Randle, (1983), Biochem., J. 88, 137).

Probe bzw. Referenzmaterial wurden zusammen mit einer vorgegebenen Menge ¹²⁵J EPO und anti-EPO Kaninchen-Antiserum 24 Stunden bei 4 °C inkubiert, dann zur Abtrennung des antikörpergebundenen ¹²⁵J EPO mit Ziege-anti-Kaninchen IgG für weitere 18 Stunden bei 4°C inkubiert. Der Niederschlag wurde abzentrifugiert, zweimal mit je 500 µl Puffer gewaschen und im Gammakanal eines automatischen Gammaspektrometers gemessen. Das System enthielt zur Verstärkung des Niederschlags gepooltes Kaninchen-Normalserum. Die Auswertung erfolgte durch Vergleich mit einer Verdünnungsreihe des Referenzmaterials anhand von Standardkurven, bei denen die prozentuale Bindung der Radioaktivität gegen den dekadischen Logarithmus der Referenzmaterialkonzentration aufgetragen wurde.

Das ¹²⁵J EPO wurde auf gereinigtem EPO durch Radiojodmarkierung nach der Zweiphasen-Chloramin-T-Methode (F. Trejedor, J.P.G. Ballestra, (1982), Anal. Biochem. 127, 143-149) gewonnen, nicht eingebautes Jodid wurde durch Gelchromatographie entfernt. Das anti-EPO-Serum wurde im Verhältnis 1:3300 vorverdünnt, das anti-Kaninchen IgG Serum 150-fach. Als Referenzmaterial diente ein Laborstandard aus reinem rekombinanten, menschlichen EPO, dessen Proteingehalt nach der BCA-Methode (P.K. Smith, (1985), Anal. Biochem. 150, 76-85) bestimmt worden war. Die Verwendung eines Antiserums gegen das gesamte EPO-Molekül gewährleistete auch den Nachweis eines EPO-Muteins.

### Enzymimmunoassay zur Identifizierung von EPO produzierenden Zellklonen.

Für das schnelle Durchprüfen (keine Verdünnung notwendig) von EPO-produzierenden Zellinien wurde ein ELISA-Dot-Blot Testsystem entwickelt. 200 µl Kulturüberstand wurden auf Nitrozellulose gesaugt. Die Filter wurden 30 Min. bei 37°C mit 0.5 % BSA in PBS pH 7.0 abgesättigt und anschließend über Nacht mit 1:1000 verdünntem anti EPO Kaninchen-Antiserum inkubiert. Nach Waschen mit PBS 0.05 % Tween erfolgte eine 2stündige Inkubation mit einem Ziege-anti-Kaninchen lg Antiserum, das mit alkalischer Phospatase gekoppelt ist. Nach weiterem Waschen mit 0.05 % Tween in PBS pH 7.0 und 0.2 M Tris-HCI ph 9,5 sowie 1 M Tris-HCI pH 9.5 werden die Substrate 4-Nitrotetrazolium-chloridblau-Hydrat und 5-Bromo-4-chloroindoxylphosphat-p-toluidinsalz zugegeben. Nach 20 Min. wird die Reaktion durch Zugabe von Wasser gestoppt. Der Test ist zum Nachweis von EPO-Mengen größer als 100 ng EPO/ml geeignet. Als Referenzmaterial dient gereinigtes rekombinantes EPO aus C127 Zellen (EP-A 267 678).

### 7) Nachweis der biologischen Aktivität von EPO und EPO Muteinen aus Kulturüberständen transfizierter und stabil exprimierender Zellen.

### in vivo Bioassay

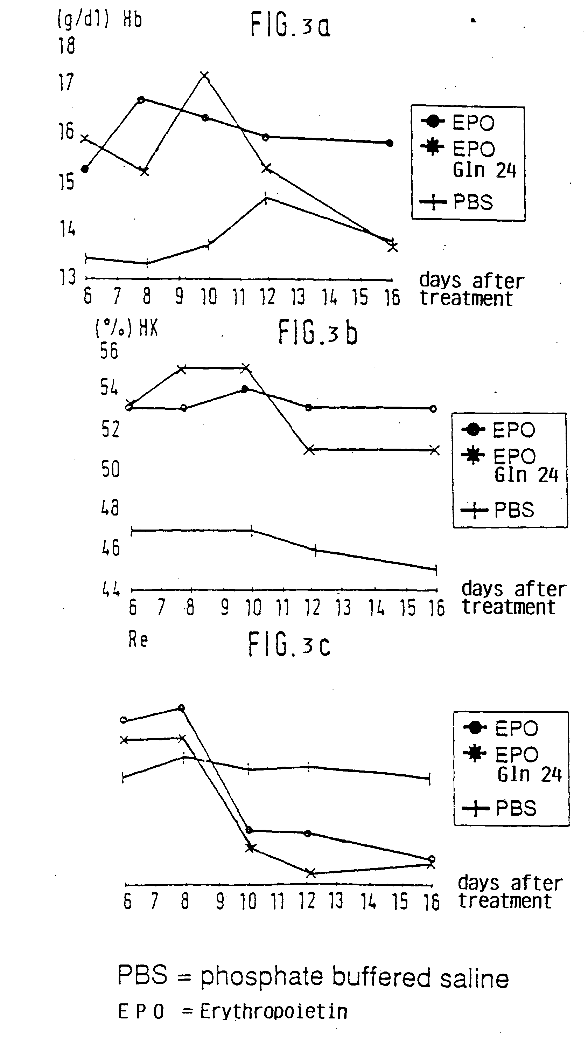

NMRI-Mäuse von der Tierzucht der Behringwerke AG (Marburg, Deutschland) wurden in randomisierte Gruppen aufgeteilt und an fünf aufeinanderfolgenden Tagen mit unterschiedlichen Dosen an EPO oder EPO-Mutein intraperitoneal mit zwei Injektionen pro Tag gespritzt. Kontrolltiere wurden mit PBS, Zellkulturmedium oder Zellkulturüberstand von BHK-Zellen behandelt. Als Referenzmaterial diente gereinigtes rekombinantes EPO aus C127 Zellen (EP-A- 267 678). Hämatologische Untersuchungen wurden an mehreren Zeitpunkten zwischen Tag 0 und Tag 22 nach Injektion der ersten EPO-Dosis durchgeführt. Sie beinhalteten die Bestimmung des Hämatokrits, des Hämoglobulingehaltes und der Retikulozytenzahl im peripheren Blut (Fig. 3a-c).

### in vitro Bioassay

Weibliche NMRI-Mäuse wurden an zwei aufeinanderfolgenden Tagen mit Phenylhydrazinhypochlorid (60 mg/ml) gespritzt. 48 Stunden nach der letzten Injektion wurden die Milzen präpariert und eine Einzelzelluspension hergestellt. Aus diesen wurden die erythroiden Vorläuferzellen über einen Ficollgradienten (D= 1.077) angereichert. Die Interphase des Gradienten wurde gesammelt, zweimal mit 20 ml PBS gewaschen, in DMEM resuspendiert und die Zellen gezählt. EPO- und EPO-Mutein-Proben wurden verdünnt und 20 µl jeder Verdünnung wurden zu je 80 µl Milzzellsuspension in eine Mikrotiterplatte gegeben (3 x 1.0⁵ Zellen/Vertiefung). Nach 22 Stunden Inkubation in feuchter Atmosphäre und 5% CO₂ wurde jeder Vertiefung 1 µCi Methyl-³H-Thymidin in 20 µl DMEM zugegeben. Die Zellen wurden 3 Stunden lang markiert, und der ³H-Einbau wurde danach in einem TRI-CARE 6660 Flüssigkeitsszintillationszähler gemessen (Krystal et al., J. Exp. Hematol. 11, 649). Referenzmaterialien waren PBS, Medium und gereinigtes, rekombinantes EPO aus C127 Zellen (EP-A- 267 678).

### 8) Isolierung von EPO und EPO Mutein produzierenden Zellklonen aus Mischklonen transfizierter BHK-Zellen

BHK-Mischklone, die aus einem Pool von 80 - 100 Einzelklonen nach Transfektion und Doppelselektion entstanden, wurden durch Immunoassays und Bioassays auf Sekretion von EPO oder EPO-Mutein untersucht. Positive Mischklone produzierten 100 ng - 1 µg EPO oder EPO-Mutein. Nach Klonierung durch ein Grenzverdünnungs-Verfahren konnten Einzelzellklone erhalten werden, die zwischen 1 bis 10 µg EPO oder EPO-Mutein produzierten. Mischklone, die weniger EPO oder EPO-Mutein produzierten, konnten über Amplifikationselektion mit Methotrexat zu vergleichbarer EPO-Produktion gebracht werden. Die biologische Aktivität in vitro (Standardisierung durch RIA bzw. ELISA) ist aus diesen ungereinigten Präparationen vergleichbar mit der Aktivität in gereinigten EPO-Fraktionen (100 U/µg - 200 U/µg). Die Muteine zeigen in vivo im Vergleich zu rhuEPO-WT unterschiedliche biologische Aktivität (Fig. 3). Um größere Mengen Zellkulturüberstände herzustellen, wurden die Einzelklone expandiert, bis sie in Rollerflaschen kultiviert werden konnten. Die Anzucht der Zellen erfolgte in serumhaltigem Medium (DMEM) bis zur Konfluenz. Dann wurde umgestellt auf serumfreies Medium (DMEM). Die Kulturüberstände wurden nach drei Tagen Kultivierung in serumfreiem Medium geerntet. Mischzellklone, die weniger EPO oder EPO-Mutein produzierten, konnten über Amplifikationsselektion mit Methotrexat zu vergleichbarer EPO-Produktion gebracht werden.

### II. Mutein EPO 7

Die N-Glykosylierungsstelle bei Asn 24 wurde durch Austausch mit GIn 24 entfernt. Die Mutante zeigt im SDS PA Gel gleiches Laufverhalten wie Wildtyp-EPO zwischen 34 und 38 kD. Im biologischen in vivo Test zeigt sich eine verzögerte Stimulierung des Retikulozytenwachstums (Fig. 3). Alle EPO 7-mischklone produzierten Mutein und es konnten Einzelklone isoliert werden, die bis zu 3.1 µg/ml/10⁶ Zellen/24 Stunden produzierten.

## Patentansprüche

1. Arzneimittel, enthaltend ein Mutein des menschlichen Erythropoietins, wobei die Aminosäure Asn 24 durch Gln 24 ersetzt ist und gegebenenfalls einen physiologisch verträglichen Träger.

2. Verwendung eines Muteins des menschlichen Erythropoietins, worin die Aminosäure Asn 24 durch GIn 24 ersetzt ist, zur Herstellung eines Arzneimittels zur Behandlung der renalen Anämie und zur Anwendung bei Therapien, die eine Erhöhung oder Erniedrigung der Erythrocytenzahl und/oder eine Erhöhung der Erythrocythenqualität zum Ziel haben.

## Claims

1. A pharmaceutical which comprises a mutein of human erythropoietin, in which the amino acid Asn 24 is replaced by Gln 24, and, where appropriate, a physiologically tolerated excipient.

2. The use of a mutein of human erythropoietin, in which the amino acid Asn 24 is replaced by Gln 24, for preparing a pharmaceutical for treating renal anemia and for use in therapies which have as their aim an increase or decrease in the erythrocyte count and/or an increase in erythrocyte quality.

## Revendications

1. Médicament, contenant une mutéine de l'érythropoïétine humaine, dans laquelle l'aminoacide Asn 24 est remplacé par Gln 24, et éventuellement un véhicule physiologiquement acceptable.

2. Utilisation d'une mutéine de l'érythropoïétine humaine, dans laquelle l'aminoacide Asn 24 est remplacé par Gln 24, pour la fabrication d'un médicament destiné au traitement de l'anémie rénale et à l'utilisation dans des thérapies qui ont pour but une augmentation ou une diminution du nombre des érythrocytes et/ou un accroissement de la qualité des érythrocytes.
